# EUROPEAN PATENT SPECIFICATION

(11) **EP 1 959 985 B1**
(45) Date of publication and mention of the grant of the patent: **14.10.2009**
(21) Application number: 06826240.1
(22) Date of filing: 19.10.2006
(51) Int. Cl.: A61K 38/22, A61P 35/00

(54) **USE OF PROLACTIN IN THE PROPHYLACTIC TREATMENT OF CANCER**
VERWENDUNG VON PROLACTIN IN DER PROPHYLAKTISCHEN KREBSTHERAPIE
UTILISATION DE LA PROLACTINE DANS LE TRAITEMENT PROPHYLACTIQUE DU CANCER

(30) Priority: 20.10.2005 US 728297 P; 01.02.2006 US 763892 P
(43) Date of publication of application: 27.08.2008
(73) Proprietor: GHC RESEARCH DEVELOPMENT CORPORATION, Greenville, SC 29605 (US)
(72) Inventor: CHEN, Wen, Yuan, Greer, SC 29650 (US)
(74) Representative: Pohlman, Sandra M.
(86) International application number: PCT/US2006/040806
(87) International publication number: WO 2007/047803

(56) References cited:
- EP-A- 0 921 193
- WO-A-99/58097
- WO-A-20/04028552
- CLEVENGER C V ET AL: "The role of prolactin in mammary carcinoma" ENDOCRINE REVIEWS, BALTIMORE, MD, US, vol. 24, no. 1, February 2003 (2003-02), pages 1-27, XP002408798

## Description

### CROSS-REFERENCE TO RELATED PATENT APPLICATIONS

This application claims priority from U.S. Provisional Application No. 60/728,297, filed 10/20/2005 and from U.S. Provisional Application No. 60/763,892, filed 02/01/2006.

### FIELD OF THE INVENTION

The present invention relates generally to prolactin containing compositions and their use in treating breast cancer.

### BACKGROUND OF THE INVENTION

Breast cancer is the second leading cause of cancer death in women in Western society. While the cause of breast cancer is still not clear, it is generally believed that tumorigenesis is not triggered by a single etiologic agent.

At present, factors known to increase a woman's chance of developing breast cancer include age as a woman's chances of getting breast cancer increase with age, family history of breast cancer in a first degree relative (mother, sister, or daughter), age of first menstrual period (the earlier the age, usually before 12 years old, the higher the risk), age at the time of menopause (the later the age, the higher the risk), use of menopausal hormone replacement drugs, and certain breast conditions.

Additionally, the incidence of breast cancer is correlated with pregnancy. For example, a woman who has her first child around age 30 has approximately the same lifetime risk of developing breast cancer as a woman who has never given birth, but a woman who has had her first child after the age of 35 has approximately twice the risk of developing breast cancer as a woman who has had a child before age 20. In other words, the younger a woman is when she has her first child, the lower her risk of developing breast cancer during her lifetime, and having more than one child at a younger age also decreases the risk.

While for decades the primary therapies for women with breast cancer have been surgery, radiation, or a combination of the two (Miller and Langdon, BIOLOGY OF FEMALE CANCERS, CRC Press LLC, pp 43-60 (1997); Forrest, BIOLOGY OF FEMALE CANCERS, CRC Press LLC, pp 31-42 (1997)), there is a low response rate to many therapies due to the heterogeneous nature of the cancer. Thus, the failure of conventional chemotherapies against advanced invasive breast cancer demands a shift of emphasis in cancer research from treatment to prevention.

The present invention satisfies that need by short-term treatment with prolactin (PRL). Although several lines of evidence have strongly linked PRL to breast cancer development, and expression levels of prolactin receptors (PRLR) reportedly are higher in human breast cancer cells than in normal breast epithelial cells (Reynolds et al., Endocrinology, 138:5555-60 (1997)) or in surgically removed breast cancer tissues (Touraine, Martini P. et al., Increased Expression Of Prolactin Receptor Gene In Human Breast Tumors Versus Continguous Normal Breast Tissues, (Abstract) 79th Annual Meeting of Endocrine Society, p.113, (1997)), this invention discloses the unexpected discovery that pre-treating a patient with prolactin reduces the risk of breast cancer.

### SUMMARY OF THE INVENTION

Accordingly, it is an object of the invention to provide a composition for preventing breast cancer, especially in a mammal which is considered at risk for the diseased comprising prolactin. Specifically, the present specification discloses (1) a method for reducing the risk of having breast cancer and (2) a method for inducing a protective response to breast cancer. Both methods comprise administering a prophylactically effective amount of prolactin to a mammal considered at risk for developing breast cancer.

In one embodiment, the mammal considered at risk for developing breast cancer is at risk of developing a Her2/neu induced breast cancer. In another embodiment, the prolactin is to be administered at very low physiological doses for short term treatment. For example, the prolactin is to be administered at a dose which achieves a serum level that is about 10 to about 20 ng/ml above basal prolactin levels.

Also described in the present specification is a method for inducing lobule differentiation in a first mammal comprising administering prolactin to a first mammal considered at risk for developing breast cancer, and, in particular, at higher risk for developing breast cancer as compared to a second mammal who carries a lower risk. In one embodiment, the prolactin is to be administered at very low physiological doses for short term treatment. For example, the prolactin is to be administered at a dose which achieves a serum level that is about 10 to about 20 ng/ml above basal prolactin levels. In another embodiment, the mammal considered at risk for developing breast cancer is at risk of developing a Her2/neu induced breast cancer.

### BRIEF DESCRIPTION OF THE DRAWINGS

Figure 1: Comparison of the breast tumor rates in female HER2/neu transgenic mice and hPRLxHER2/neu bi-transgenic mice.

Figure 2: Images of the fourth inguinal mammary glands of nulliparous mice after staining with Carnoy's stain.

Figure 3A and 3B: DNA and amino acid sequences of a peptide modified prolactin, PRL-SA20.

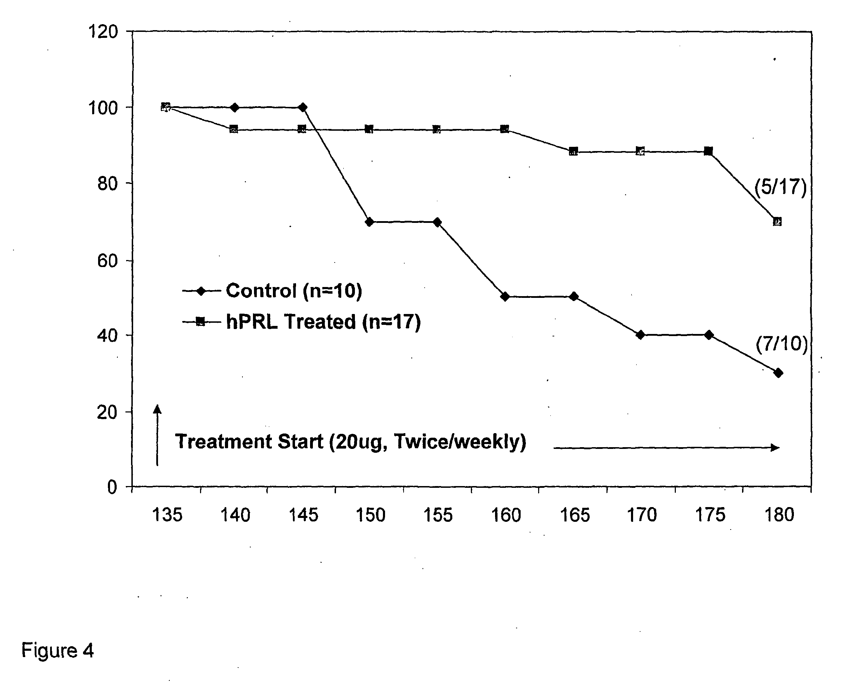

Figure 4: Graph of tumor rates in MMTV-HER2/neu transgenic mice in prolactin treated and untreated animals.

### DETAILED DESCRIPTION OF THE PREFERRED EMBODIMENTS

The present invention discloses the unexpected discovery that short term prolactin treatment reduces the incidence of breast cancer in a mammal considered at risk for the disease. This is surprising since several lines of evidence have strongly linked prolactin to breast cancer development and, therefore, prolactin would not be predicted to elicit a protective effect on the breast. For example, prolactin has been shown to be produced locally by some breast cancer cells (Ginsburg et al., Cancer Res., 55:2591-5 (1995) and Clevenger et al., J. Mammary Gland Biol. Neo., 2:59-68 (1997)), prolactin receptor expression levels have been shown to be upregulated in breast cancer (Reynolds et al. (1997) and Touraine et al., J. Clin., Endocrinol. Metab., 83:667-74 (1998)), and prolactin has been shown to act as an anti-apoptotic agent and anti-prolactin agents have been shown to induce apoptosis in breast cancer cells (Chen et al., Clin. Cancer Res. 5:3583-93 (1999)). Accordingly, the present invention describes prophylactic treatment methods for candidates who are within a risk population for breast cancer.

As described herein, a mammal considered to be at risk for developing breast cancer is one who has a greater likelihood of developing the disease because he or she possesses a particular risk factor, than a mammal who does not have that particular risk factor. Risk factors include, but are not limited to, a family history of breast cancer, especially a HER2 positive breast cancer, early age of first menstrual period, especially if earlier than 12 years of age, nulliparity, older age at the time of menopause, age at the time of first pregnancy, especially if older than 24 years of age, post-menopausal obesity, and/or not breast-feeding.

HER2/neu (human epidermal growth factor receptor 2) is a gene that when expressed, helps control how cells grow, divide, and repair themselves, important in the control of abnormal or defective cells that could become cancerous. About 30 percent of all breast cancer tumors are HER2/neu positive.

The prolactin for use in the present invention includes wild-type prolactin as well as conservative variants thereof. The overall structure and composition of the prolactin is important only insofar it confers the appropriate functional characteristics, i. e., prolactin receptor binding. A skilled artisan would understand that typically the prolactin for use as described herein should be species matched with the mammal being treated. For example, a human being treated with prolactin should typically be treated with a human prolactin.

A prolactin variant according to the present invention generally conserves the overall molecular structure of the protein. Given the properties of the individual amino acids in prolactin, some rational substitutions will be apparent. Amino acid substitutions, *i.e*. "conservative substitutions," may be made, for instance, on the basis of similarity in polarity, charge, solubility, hydrophobicity, hydrophilicity, and/or the amphipathic nature of the residues involved. Such conservative variants of prolactin retain two functional prolactin receptor binding sites and are able to initiate the prolactin signaling cascade.

For example: (a) nonpolar (hydrophobic) amino acids include alanine, leucine, isoleucine, valine, proline, phenylalanine, tryptophan, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt α-helices. Similarly, certain amino acids, such as alanine, cysteine, leucine, methionine, glutamic acid, glutamine, histidine and lysine are more commonly found in α-helices, while valine, isoleucine, phenylalanine, tyrosine, tryptophan and threonine are more commonly found in β-pleated sheets. Glycine, serine, aspartic acid, asparagine, and proline are commonly found in turns. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants.

Conservative variants of the present invention specifically contemplate truncations of the presently described prolactin. Truncations may be made from the Nor C-terminus, but generally do not entail deleting more than about 30% of the native molecule. In one embodiment, less than about 20%, less than about 10%, less than about 5%, or less than about 2% of the native molecule is deleted. The term "about" with regard to percentage of protein truncated connotes +/- 2%. For example, deleting about 10% of the native molecules refers to a 8%-12% truncation of the native molecule. Again, so long as the prolactin (wild-type and variant prolactin) confers the appropriate functional characteristics, *i.e.,* prolactin receptor binding and receptor dimerization and signal transduction, then it is suitable for use in the present invention.

Other prolactin variants suitable for use in the present invention are prolactin multimers, *i.e*., homo and heteromultimers of prolactin (including wild-type prolactin and prolactin variants) that have at least two active receptor binding sites so as to still permit receptor dimerization and signal transduction to occur. For example, a prolactin homodimer may comprise two prolactin antagonists such as G129-hPRL. Although each monomer individually is a prolactin antagonist, the homodimer acts as a prolactin agonist. In another embodiment, the prolactin multimer is a heterodimer, which comprises a prolactin antagonist (such as G129R-hPRL) and a prolactin agonist (such as wild-type prolactin). Thus, the heterodimer would be a hPRL-hPRL-G129R heterodimer.

Cell-based assay systems can be used to compare the biological activities of a wild-type prolactin and a prolactin variant. To this end, competitive binding assays may be used to ensure that each prolactin variant binds to its normal receptor and competes with wild-type prolactin for receptor binding. Competitive binding assays are routine and known in the art.

Prolactin variant activity can also be readily assessed by phosphorylation assays (e.g., immunoprecipitation assays), which are known in the art. Cell signaling via wild-type prolactin is initiated by prolactin binding to the prolactin receptor. This binding induces dimerization of the prolactin receptor, thereby triggering a signal transduction cascade in cells comprising a prolactin bound receptor. Signal transduction in the prolactin signaling pathway involves signal transducers and activators of transcription (STAT) phosphorylation, and therefore, STAT phosphorylation assays can be used to determine whether the prolactin variant is suitable for use in the present invention.

In general, the prolactin of the present invention, and DNA encoding the prolactin, can be defined with reference to "sequence identity." Some prolactin variants have at least about 50%, at least about 55% or at least about 60% sequence identity to a wild-type prolactin. Others have at least about 65%, at least about 70%, at least about 80%, at least about 85%, at least about 90%, at least about 95%, at least about 98% or at least about 99% sequence identity to a wild-type prolactin. The term "about" with respect to a specific percentage sequence identity connotes +/- 2%. For example, the term "about 95% sequence identity" refers to a range of 93%-97% sequence identity. As used herein, two nucleic acid molecules or proteins are said to "share significant sequence identity" if the two contain regions possess greater than 85% sequence (amino acid or nucleic acid) identity.

"Sequence identity" is defined herein with reference to the Blast 2 algorithm, which is available at the NCBI (http://www.ncbi.nlm.nih.gov/BLAST), using default parameters. References pertaining to this algorithm include: those found at http://www.ncbi.nlm.nih.govBLAST/blast_references.html; Altschul et al., (1990) "Basic local alignment search tool." J. Mol. Biol. 215: 403-410; Gish, W. & States, D.J. (1993)"Identification of protein coding regions by database similarity search." Nature Genet. 3: 266-272; Madden et al. (1996) "Applications of network BLAST server" Meth. Enzymol. 266: 131-141; Altschul et al., (1997) "Gapped BLAST and PSI-BLAST: a new generation of protein database search programs." Nucleic Acids Res. 25: 3389-3402; and Zhang, J. & Madden, T.L. (1997) "PowerBLAST: A new network BLAST application for interactive or automated sequence analysis and annotation." Genome Res. 7: 649-656. Accordingly, the prolactin peptide sequences from different species, which include those listed in Table 1, can be aligned using standard computer programs like BLAST to inform further variation in prolactin-derived receptor-antagonizing domains that preserve their essential function.

The prolactin of the present invention (*i.e*., wild-type and prolactin variants) can be prepared by chemical synthesis or recombinant DNA techniques. Generally, a cDNA of prolactin may be prepared using standard PCR amplification techniques, RNA or cDNA prepared from a cell which produces prolactin (such as a pituitary cell) as a template, and oligonucleotide primers designed based on known prolactin nucleic acid or amino acid sequence. A non-limiting example of the preparation of a cDNA encoding human prolactin is set forth in published U.S. Patent Application No. 2003 0022833 to Wagner et al*.* Alterations may then be introduced into the prolactin cDNA either randomly or by directed mutagenesis. An example of the use of oligonucleotide mediated site-directed mutagenesis is also set forth in published U.S. Patent Application No. 2003 0022833 to Wagner et al*.*

Where the prolactin is to be produced by recombinant techniques, a nucleic acid encoding prolactin may be incorporated into an expression vector, operatively linked to a suitable promoter/enhancer sequence. The expression vector may further contain one or more elements which aid in the expression of prolactin, including a transcription termination site, a polyadenylation site, a ribosome binding site, a signal sequence, etc. Suitable expression systems include mammalian cells, insect cells, plant cells, yeast cells, slime mold, and organisms, including transgenic plants and transgenic animals. Suitable expression vectors include herpes simplex viral based vectors such as pHSV1 (Geller et al., Proc. Natl. Acad. Sci. U.SA. 87:8950-8954 (1990)); retroviral vectors such as MFG (Jaffee et al., Cancer Res. 53:2221-2226 (1993)), and in particular Moloney retroviral vectors such as LN, LNSX, LNCX, LXSN (Miller and Rosman, Biotechniques, 7:980-989 (1989)); vaccinia viral vectors such as MVA (Sutter and Moss, Proc. Natl. Acad. Sci. U.S.A. 89:10847-10851 (1992)); adenovirus vectors such as pJM17 (Ali et al., Gene Therapy 1:367-384 (1994); Berker, Biotechniques 6:616-624 (1988); Wand and Finer, Nature Medicine 2:714-716 (1996)); adeno-associated virus vectors such as AAV/neo (Mura-Cacho et al., J. Immunother., 11:231-237 (1992)); lentivirus vectors (Zufferey et al., Nature Biotechnology 15:871-875 (1997)); plasmid vectors such as pCDNA3 and pCDNA1 (In Vitrogen), pET 11a, pET3a, pET11d, pET3d, pET22d, and pET12a (Novagen); plasmid AH5 (which contains the SV40 origin and the adenovirus major late promoter), pRC/CMV (InVitrogen), pCMU II (Paabo et al., EMBO J., 5:1921-1927 (1986)), pZipNeo SV (Cepko et al., Cell, 37:1053-1062 (1984)), pSR.alpha. (DNAX, Palo Alto, Calif.) and pBK-CMV; and baculovirus expression vectors (O'Reilly et al., BACULOVIRUS EXPRESSION VECTORS, Oxford University Press (1995)), such as p2Bac (In Vitrogen).

The prolactin for use in the present invention can also be modified to have a longer clearance rate and therefore increase bioavailability by protecting the composition from an immune response and other clearance mechanisms afforded by the subject. PEG (polyethylene glycol)-ylated compounds exhibit reduced immunogenicity and antigenicity, and circulate in the bloodstream considerably longer than unconjugated proteins. Thus, to increase prolactin half-life, the present invention contemplates attaching a PEGpolymer chain to prolactin. This can be accomplished by methods known in the art, such as by the PEGylation procedure described in Roberts et al., Adv. Drug Del. Rev., 54(4):459-76 (2002). In addition, other agents which can prolong elimination half-life of prolactin are known to the skilled artisan and are contemplated herein.

For example, the prolactin described herein can also be chemically modified by techniques known in the art to increase serum half-life of a protein. For example, prolactin can be modified with hydroxyethylstarch (HES), which is a derivative of naturally occurring amylopektine that is degraded by α-amylase in the body. Thus, in one embodiment, wild-type prolactin or a prolactin variant can be "hesylated". Examples of HES compounds include, but are not limited to, a 10 kD or 50 kD HES. Methods for making HES-protein conjugates are known in the art. *See, for example,* EP 1398322, DE 2616086 and DE 2646854.

In addition, agents that link prolactin to serum albumin are also contemplated in the present invention to prolong clearance time and increase half-life of the inventive composition. While such agents are disclosed in WO 01/45746,
other peptide ligands which have an affinity for serum albumin that can be conjugated to prolactin are also considered suitable for use herein.

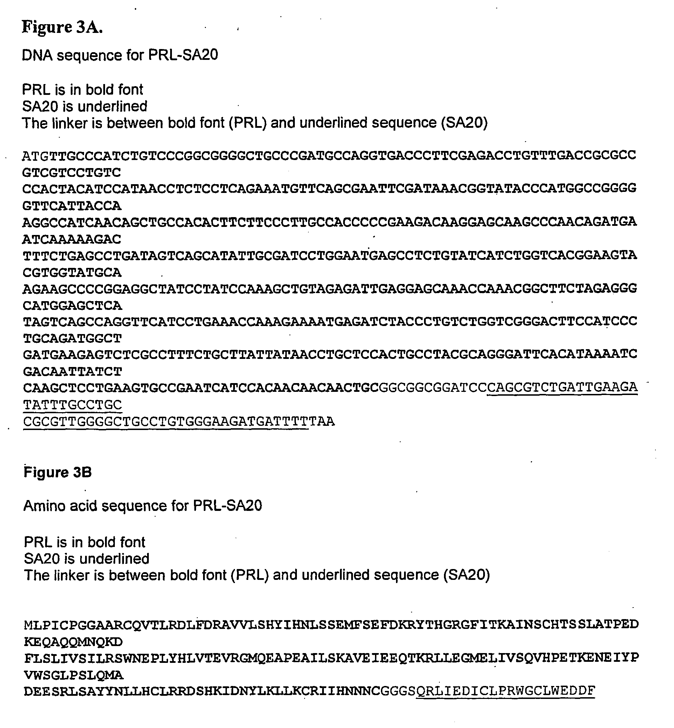

Also, prolactin (wild-type and prolactin variants) may be modified by, for example, the addition of a peptide sequence. Such peptide modifications increase serum half-life of the prolactin and can be of any length so long as the serum half life of the protein is increased relative to a protein that does not contain the additional peptide sequence modification. Exemplary additional peptide sequence lengths include, but are not limited to, at least about 40, about 35, about 34, about 33, about 32, about 31, about 30, about 29, about 28, about 27, about 26, about 25, about 24, about 23, about 22, about 21, about 20, about 19, about 18, about 17, about 16, about 15, about 14, about 13, about 12, about 11, or about 10 amino acids in length. See, for example, Figure 3 and Dennis et al., J. Biol. Chem., 277:35035-43 (2002).
As described herein, serum half life can be measured by techniques known in the art. The peptide sequence can be added to the N or C terminus of the protein.

The proteins of the present invention can be formulated according to known methods to prepare pharmaceutically useful compositions, whereby the inventive molecules, or their functional derivatives, are combined in admixture with a pharmaceutically acceptable carrier vehicle. Suitable vehicles and their formulation are described, for example, in Remington's Pharmaceutical Sciences (16th ed., Osol, A., Ed., Mack, Easton PA (1980)). To form a pharmaceutically acceptable composition suitable for effective administration, such compositions will comprise prolactin and optionally, another protein or active agent, together with a suitable amount of carrier vehicle or excipient. Thus, the pharmaceutical composition of the present invention may be formulated for administration by inhalation or insufflation (either through the mouth or the nose), parenteral injection (*e.g*., intravenous, intramuscular, or subcutaneous), oral administration in solid, liquid, or aerosol form, buccal, rectal, vaginal, nasal, ocular, local (powders, ointments or drops), intracisternal, intraperitoneal, or topical administration, and the like.

For oral administration, the pharmaceutical compositions may take the form of, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g*., lactose, microcrystalline cellulose or calcium-hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they maybe presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the composition may take the form of tablets or lozenges formulated in conventional manner.

For administration by inhalation, the composition for use according to the present invention can be conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g*. gelatin for use in an inhaler or insufflator may be formulated containing a powder mix of the compound and a suitable powder base such as lactose or starch.

The compositions described herein may be formulated for parenteral administration by injection, *e.g*., by bolus injection or continuous infusion. Formulations for injection may be presented in unit dosage form, *e.g*., in ampules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

The compounds may also be formulated in rectal compositions such as suppositories or retention enemas, *e.g*., containing conventional suppository bases such as cocoa butter or other glycerides.

In addition to the formulations described previously, the compositions of the present invention may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

Accordingly disclosed herein is a prophylactic method for reducing the risk of breast cancer in a mammal comprising administering a prophylactically effective amount of prolactin to a mammal considered at risk for developing breast cancer. In another aspect, the prolactin is administered to a mammal having a higher risk as compared to a mammal having a lower risk of breast cancer. In one aspect, a mammal considered at risk, or at higher risk, for developing breast cancer is a mammal who possesses a risk factor known to increase a mammal's chance of developing breast cancer. Such risk factors include, but are not limited to, a family history of breast cancer, especially a HER2 positive breast cancer, early age of first menstrual period, especially if earlier than about 12 years of age, nulliparity, older age at the time of menopause, age at the time of first pregnancy, especially if older than about 24 years of age, post-menopausal obesity, and/or not breast-feeding.

The prophylactic treatment methods described herein may be suitable for a mammal of any age who is at risk for developing breast cancer. Thus, a mammal may be prophylactically treated with prolactin at any age prior to the onset of disease, and the onset of disease can be determined by methods known to those of skill in the art. As an example, the prolactin can be administered, for example, to a mammal at least about 15, at least about 17, at least about 18, at least about 19, at least about 20, at least about 21, at least about 22, at least about 23, at least about 24, at least about 25, or at least about 26 years of age. Indeed, a mammal may be prophylactically treated with prolactin (wild-type and variants) for breast cancer when the mammal is less than about 50, less than about 45, less than about 40, less than about 35, less than about 30, less than about 25, less than about 24, less than about 23, less than about 22, less than about 21, less than about 20, less than about 19, less than about 18, less than about 17, less than about 16, less than about 15, less than about 14, less than about 13, less than about 12, or less than about 10 years of age.

According to the present invention, the frequency in which prolactin is administered may be periodic, such as daily, every other day, every third day, or weekly, depending on the serum half-life of the prolactin. One of skill in the art would be able to determine a prophylactically effective amount of prolactin based on the guidance provided herein.

The propylactically effective amount of prolactin can be administered on a short term basis. Exemplary short term periods of administration include, but are not limited to, less than about 42 weeks, less than about 40 weeks, less than about 36 weeks, less than about 32 weeks, less than about 28 weeks, less than about 24 weeks, less than about 20 weeks, less than about 16 weeks, less than about 12 weeks, less than about 8 weeks, less than about 4 weeks, or less than about 2 weeks. In one embodiment, the mammal considered at risk for developing breast cancer is at risk of developing a Her2/neu induced breast cancer.

"Prophylactically effective" is employed herein to denote the amount of prolactin that is of sufficient quantity to prevent, delay onset, or reduce the risk of developing breast cancer, and in particular, a Her2 breast cancer. A risk of developing breast cancer is reduced when the likelihood of a breast cancer diagnosis is less after prolactin administration than before prolactin administration. For example, a prophylactically effective amount of prolactin is the amount of prolactin necessary to achieve a serum level of less than about 5 to about 60 ng/ml, less than about 10 to about 50 ng/ml, less than about 15 to about 50 ng/ml or less than about 10 to about 20 ng/ml above basal levels. The term "about" with reference to serum levels of prolactin connotes +/- 2 ng/ml. Therefore, the phrase "less than about" 10-20 ng/ml means less than 8-22 ng/ml.

Also described herein is a method of reducing the incidence of breast cancer comprising exposing mammary epithelial cells of a Lob 1 or a Lob 2 lobule type to prolactin. Lob 1 refers to an undifferentiated type of lobule in breast tissue in normally cycling women and Lob2 refers to a more differentiated or developed lobule in breast tissue.

A prophylactically effective amount can also refer to the amount of prolactin which can induce lobule differentiation, such as physiological concentrations of prolactin which mimic prolactin levels during early pregnancy. For example, relatively low prolactin concentrations, such as serum concentrations of less than about 50 ng/ml, less than about 30 ng/ml, less than about 20 ng/ml, or even less than about 10 ng/ml above basal levels can be prophylactically effective.

In general, a prophylactically effective amount of the compositions described herein largely will depend on particular patient characteristics, route of administration, and the nature of the disorder being treated. General guidance can be found, for example, in the publications of the International Conference on Harmonisation and in REMINGTON'S PHARMACEUTICAL SCIENCES, chapters 27 and 28, pp. 484-528 (Mack Publishing Company 1990).

Determining a prophylactically effective amount will also depend on such factors as toxicity and efficacy of the medicament, including half-life of the protein. Toxicity may be determined using methods well known in the art and found in the foregoing references. Efficacy may be determined utilizing the same guidance in conjunction with the methods described below in the Examples. A prophylactically effective amount will be an amount that is deemed by the clinician to be toxicologically tolerable, yet efficacious. Suitable dosages are those which achieve are those which achieve a serum concentration of less than about 50 ng/ml, less than about 30 ng/ml, less than about 20 ng/ml, or less than about 10 ng/ml above basal levels.

The prolactin-containing compositions of the present invention may be formulated in conjunction with other cancer prevention agents including, but not limited to human chorionic gonadotropin (HCG) and other agents that can induce mammary gland differentiation. For example, other hormones that can induce mammary gland differentiation/proliferation include hormones that induce mammary gland differentiation in a pregnant animal. Thus, estrogen and/or progesterone may be used in combination with prolactin to prophylactically treat a breast cancer.

As discussed herein, administration during *in vivo* treatment may be by any number of routes, including parenteral and oral, but preferably parenteral. Intracapsular, intravenous, intrathecal, and intraperitoneal routes of administration may be employed, generally intravenous is preferred. The skilled artisan will recognize that the route of administration will vary depending on the disorder to be treated.

The following examples are given to illustrate the present invention. It should be understood, however, that the invention is not to be limited to the specific conditions or details described in these examples.

### EXAMPLES

### Example 1: PRL/HER2/neu bi-transgenic mice exhibited a delayed tumor response

The purpose of this experiment was to determine if prolactin/HER2/neu transgenic mice have a delayed tumor response.

Prolactin has been suggested to be involved in breast cancer initiation/development as autocrine/paracrine factor. HER2/neu transgenic mice express the HER2/neu activated oncogene, and are a mouse model of breast cancer that is much more similar to human disease than transplanted tumors. The phenotypes of MMTV/neu homozygous transgenic mice were reported with a median incidence of mammary tumor at 205 days and >90% of the mice develop a tumor after one year (Guy et al., Proc. Natl. Acad. Sci., U.S.A., 89:10578-82 (1992)).

Human PRL transgenic mice (MT-hPRL) (Pierce S and Chen WY, Oncogene, 23:1248-1255 (2004), and Chen et al., Proc. Natl. Acad. Sci., U.S.A., 87, 5061-5065 (1990)) were cross-bred with homozygote mice of HER2/neu (The Jackson Laboratory, Bar Harbor, ME) to generate hPRLxHER2/neu bi-transgenic mice (heterozygotes for both HER2/neu and hPRL).

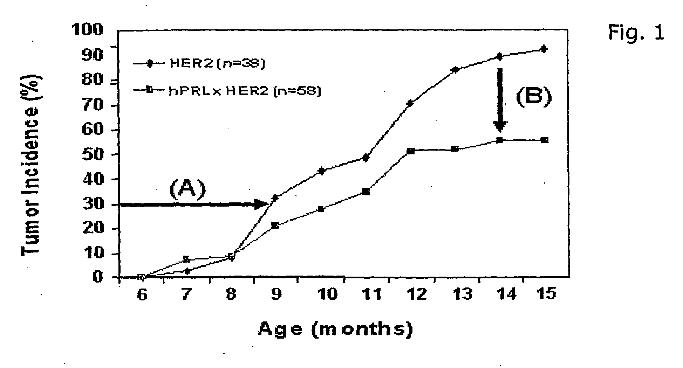

Results showed an initial delay in tumor appearance: 11 months for 30% bi-transgenic mice to developed breast tumor as compared to 9 months in the HER2/neu heterozygote littermates. See Figure 1, arrow A.

In addition, Figure 1 demonstrates that the total tumor incidence for the hPRLxHER2/neu bi-transgenic mice was reduced to approximately 50% at the age of 12 months or older (arrow B), whereas there was a steady increase in the tumor incidence in HER2/neu transgenic mice which ultimately reached >90%.

### Example 2. hPRL acts as a chemopreventive agent by reducing tumor rate

hPRL is used as a chemopreventitive agent to delay the onset and ultimately reduce the breast tumor rate in Her2/neu transgenic mice.

### Protein Production and Purification

Human PRL is produced in *E.coli* according to the published protocols (Beck et al., Oncogene, 21:5047-55 (2002)). Briefly, BL21 (DE3) cells (Invitrogen) are transformed with pET22b plasmids (Novagen, Madison, WI) which contain cDNAs encoding the hPRL. A seed culture is grown overnight at 37°C, and on the following day, a large-scale LB growth culture is prepared and IPTG is added to induce expression ofhPRL. Bacteria are collected, resuspended in a solution containing 0.2M NaPO₄ pH 8, 10mM EDTA, and 0.5% Triton X-100, and are lysed using a Sonic Dismembrator. The inclusion bodies are collected and resuspended in 0.2M NaPO₄ pH7, 1%v/v beta mercaptoethanol, and 8M urea for refolding. The refolding process consists of dialyzing the protein against decreasing amounts of urea and beta-mercaptoethanol in the presence of 20mM Tris HCl, pH 8.0 for three consecutive days. The sample is then purified by a Q-Sepharose anionic exchange column using a FPLC system. Based on previous experience, the purity of the products is typically 98% pure, as determined by SDS-PAGE and silver staining. The endotoxin level in the final products in most cases is <5 EU/mg (tested by Cape Cop Inc.).

### Dosing and Duration of hP RL Delivery

Three types of Alzet pumps are tested. The internal space (volume for drug) is 100 µl of each of the pumps. The rates of release for the 3 pumps are 0.25 µl/hr for the two week pump, 0.5 µl/hr for one week and 1 µl/hr for three days. The two week releasing pump is used in this study to minimize the number of surgeries and also maintain effective serum drug concentration. The hPRL protein concentrations after purification are optimized to -5 mg/ml, which translates to 0.5mg/pump/mouse for 2 weeks. Based upon preliminary experiments, the serum hPRL concentration is maintained at an average concentration of >50 ng/ml, which is above the serum concentration of the hPRL transgenic mice.

hPRL treatment starts at one month of age. Two mice are sacrificed and the mammary whole mount and Western blot analyses are conducted. Samples for microarray studies are collected at the end of 11 months from all groups except 7) and 9). T50 and T80 are the 50% or 80% tumor rates of untreated homozygotic Her2/neu mice at 7 months or 10 months of age, respectively.

Animals are examined twice weekly for any tumor appearance (the date of first appearance will be recorded) and the number of tumors per mice are compared between groups. Tumor growth (size) is measured twice weekly and is plotted as growth curves as previous reported (Chen et al., Int. J. Oncology, 20:813-8 (2002)). Tumors from different groups are dissected and stored at -80 °C for biomarker analyses (Western blotting). It is predicted that tumor growth will be delayed or tumor size will be reduced in hPRL treated Her2/neu mice, compared to untreated animals.

### Example 3.

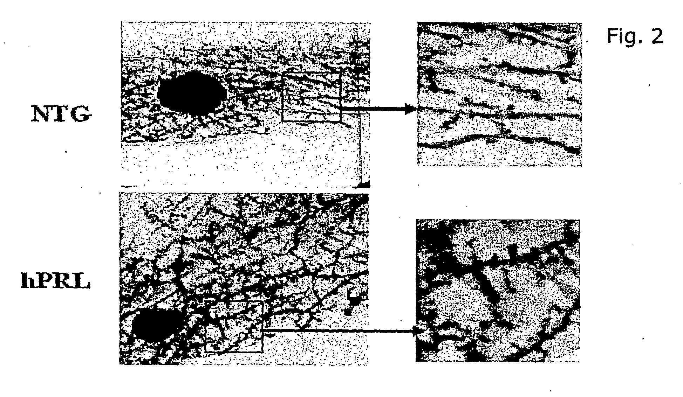

Whole mounts of the fourth inguinal mammary gland of nulliparous mice were stained with Carnoy's stain. As demonstrated in figure 2, an increase in budding (proliferation/differentiation) was observed in the mammary gland of hPRL transgenic mice. This suggests that early exposure of the mammary gland to physiological concentrations of hPRL induced lobule differentiation and that exposure may lead to a refractory state of the mammary epithelium to the HER2/neu oncogene.

### Example 4.

MMTV-HER2/neu mice (The Jackson Laboratory, Bar Harbor, ME) were administered a low dose of peptide modified human prolactin or phosphate buffered saline (PBS). In particular, the animals were given either 20 µg/mouse/twice weekly, 100 µl per injection hPRL-SA20 (figure 3), or 100 µl PBS twice weekly prior to tumor onset, at approximately 5 months of age. Results indicated that 7/10 control mice developed tumors, compared to 5/17 treated mice after 5 weeks of prolactin treatment.

### ILLUSTRATED ASPECTS

Additional aspects are disclosed. For example, the invention is further illustrated by the following numbered aspects:
1. A method for reducing the risk of having breast cancer, comprising administering a prophylactically effective amount of prolactin to a mammal considered at risk for developing breast cancer.
2. The method of aspect 1, wherein the mammal considered at risk for developing breast cancer is at risk of developing a Her2/neu induced breast cancer.
3. The method of aspect 1, wherein the prolactin is administered at very low physiological doses for short term treatment.
4. The method of aspect 1, wherein the prolactin is administered at a dose which achieves a serum level that is 10-20 ng/ml above basal prolactin levels.
5. A method for inducing lobule differentiation in a first mammal comprising administering prolactin to a first mammal considered at risk for developing breast cancer.
6. The method of aspect 5, wherein the prolactin is administered at very low physiological doses for short term treatment.
7. The method of aspect 5, wherein the prolactin is administered at a dose which achieves a serum level that is 10-20 ng/ml above basal prolactin levels.
8. The method of aspect 5, wherein the mammal considered at risk for developing breast cancer is at risk of developing a Her2/neu induced breast cancer.
9. A method for inducing a protective response to breast cancer comprising administering prophylactically effective amount of prolactin to a mammal considered at risk for developing breast cancer.
10. The method of aspect 9, wherein the prolactin is administered at very low physiological doses for short term treatment.
11. The method of aspect 9, wherein the prolactin is administered at a dose which achieves a serum level that is 10-20 ng/ml above basal prolactin levels.
12. The method of aspect 9, wherein the mammal considered at risk for developing breast cancer is at risk of developing a Her2/neu induced breast cancer.
13. A method of reducing the incidence of breast cancer comprising exposing mammary epithelial cells of a Lob 1 undifferentiated lobule type to prolactin.
14. The method of aspect 13, wherein the prolactin is administered at very low physiological doses for short term treatment.
15. A method of reducing the incidence of breast cancer comprising exposing mammary epithelial cells of a Lob2 less differentiated lobule type to prolactin.
16. The method of aspect 15, wherein the prolactin is administered at very low physiological doses for short term treatment.

## Claims

1. A composition comprising prolactin for use in
(a) reducing the risk of having breast cancer, or
(b) inducing a protective response to breast cancer.

2. Use of a composition comprising prolactin for the preparation of a pharmaceutical composition for
(a) reducing the risk of having breast cancer, or
(b) inducing a protective response to breast cancer.

3. The use of claims 1 or 2, wherein the prolactin induces lobule differentiation in a mammal considered at risk for developing breast cancer.

4. The use of any one of the preceding claims, wherein the prolactin is to be administered at a dose which achieves a serum level that is 10-20 ng/ml above basal prolactin levels.

5. The use of any one of the preceding claims, wherein the breast cancer is a Her2/neu induced breast cancer.

6. A composition comprising prolactin for use in reducing the incidence of breast cancer comprising exposing mammary epithelial cells of
(a) a Lob1 undifferentiated lobule type, or
(b) a Lob2 more differentiated lobule type
to prolactin.

7. The use of any one of the preceding claims, wherein the prolactin is to be administered at very low physiological doses for short term treatment.

## Patentansprüche

1. Zusammensetzung umfassend Prolactin zur Verwendung bei
(a) der Verminderung des Brustkrebsrisikos; oder
(b) der Auslösung einer Schutzreaktion gegen Brustkrebs.

2. Verwendung einer Zusammensetzung umfassend Prolactin zur Herstellung einer pharmazeutischen Zusammensetzung zur
(a) Verminderung des Brustkrebsrisikos; oder
(b) Auslösung einer Schutzreaktion gegen Brustkrebs.

3. Verwendung nach einem der Ansprüche 1 oder 2, wobei das Prolactin lobuläre Differenzierung bei einem Säugetier auslöst, bei dem das Risiko besteht, dass es Brustkrebs bekommt.

4. Verwendung nach einem der vorhergehenden Ansprüche, wobei das Prolactin in einer Dosis zu verabreichen ist, die einen Serumspiegel erreicht, der 10-20 ng/ml über dem Grundspiegel von Prolactin liegt.

5. Verwendung nach einem der vorhergehenden Ansprüche, wobei es sich bei dem Brustkrebs um einen durch Her2/neu ausgelösten Brustkrebs handelt.

6. Zusammensetzung umfassend Prolactin zur Verwendung bei der Verminderung der Inzidenz von Brustkrebs, umfassend Epithelzellen der Brust
(a) eines undifferenzierten lobulären Typs Lob1, oder
(b) eines stärker differenzierteren lobulären Typs Lob2
Prolactin auszusetzen.

7. Verwendung nach einem der vorhergehenden Ansprüche, wobei das Prolactin in sehr geringen physiologischen Dosen zur Kurzzeitbehandlung zu verabreichen ist.

## Revendications

1. Composition comprenant de la prolactine pour utilisation dans
(a) la réduction du risque d'atteinte du cancer du sein, ou
(b) l'induction d'une réponse protectrice au cancer du sein.

2. Utilisation d'une composition comprenant de la prolactine pour la préparation d'une composition pharmaceutique pour
(a) la réduction du risque d'atteinte du cancer du sein, ou
(b) l'induction d'une réponse protectrice au cancer du sein.

3. Utilisation des revendications 1 ou 2, dans laquelle la prolactine induit une différenciation de lobules chez un mammifère considéré comme susceptible de développer un cancer du sein.

4. Utilisation selon l'une quelconque des revendications précédentes, dans laquelle la prolactine doit être administrée à une dose qui réalise un taux de sérum supérieur de 10-20 ng/ml aux taux de prolactine basale.

5. Utilisation selon l'une quelconque des revendications précédentes, dans laquelle le cancer du sein est un cancer du sein induit par Her2/neu.

6. Composition comprenant de la prolactine pour utilisation dans la réduction de l'incidence du cancer du sein, comprenant l'exposition de cellules épithéliales mammaires d'un
(a) type de lobule non-différencié Lob1, ou
(b) d'un type de lobule plus différencié Lob2 à la
prolactine.

7. Utilisation selon l'une quelconque des revendications précédentes, dans laquelle la prolactine doit être administrée à des très faibles doses physiologiques pendant un traitement de courte durée.
